# EUROPEAN PATENT APPLICATION

(11) **EP 1 939 166 A1**
(43) Date of publication of application: **02.07.2008**
(21) Application number: 06026244.1
(22) Date of filing: 19.12.2006
(51) Int. Cl.: C07C 47/542, A61K 31/11, A61P 25/28

(54) **Plants extracts for use in brain modulation**

(71) Applicant: InterMed Discovery GmbH, 44227 Dortmund (DE)
(72) Inventor: Roemer, Ernst Dr., 07751 Bucha (DE); Grothe, Torsten Dr., 51063 Köln (DE)
(74) Representative: Maucher, Wolfgang

(57) **Abstract**

Compounds obtainable from plants, e.g. of the genus *Eucalyptus,* or from microorganisms are shown to be useful as CNS activity modulators useful e.g. in the treatment of depression, for lifting mood and/or for increasing behavioural initiative and the like. This use and related aspects form embodiments of the invention. Also compounds as such are presented.

The compounds useful are acylphloroglucine derivatives of the formula I, 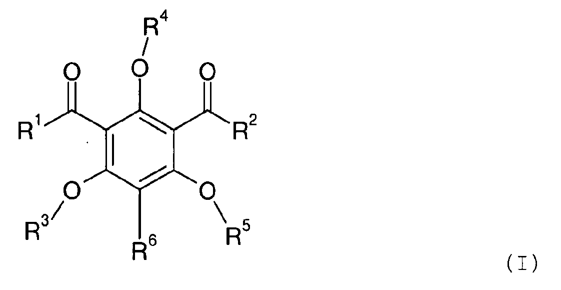 wherein the substituents are as defined in the description.

## Description

The invention relates to natural compounds or derivatives of them, to compositions comprising them, and/or to their use as modulating agents in several brain functions in humans or other animals.

Eucalyptus contains many chemical compounds that play several roles in the plant. These include defence against insect and vertebrate herbivores and protection against UV radiation and against cold stress. The best-known compounds are the terpenoids, which form most of the essential oil giving Eucalyptus foliage its characteristic smell. However, Eucalyptus is also a rich source of phenolic constituents such as tannins and simpler phenolics. Some of these have formed the basis of industries in the past. For example, tannins were extracted from Eucalyptus astringens and rutin from *E. macrorhyncha* (Lassak EV and McCarthy T 1992 Australian Medicinal Plants. Mandarin, Port. Melbourne).

However, the most recent interest in phenolic compounds from Eucalyptus has focused on a newly identified group called the formylated phloroglucinol compounds (FPCs) (Lawler IR, Foley WJ, Eschler B.M 2000 Foliar concentration of a single toxin creates habitat patchiness for a marsupial folivore. Ecology 81:1327-1338). This grouping includes the subtypes known informally as euglobals, macrocarpals and sideroxylonals. All FPCs have the same fully substituted, formylated, aromatic moiety, but vary in the structure of the side chain. In macrocarpals and euglobals the sidechain is a C10 or C15 unit derived from common foliar terpenes such as bicyclogermacrene, pinene or phellandrene but in the sideroxylonals and the simple FPCs (e.g. jensenone), the side chain is a C5 unit (Ghisalberti EL 1996 Bioactive acylphloroglucinol derivatives from Eucalyptus species, Phytochem, 41:7-22).

Formylated phloroglucinol compounds have a wide range of biological actions such as antifouling properties (Singh I P, Umehara K, Etoh H. Macrocarpals in Eucalyptus SPP. as attachment-inhibitors against the blue mussel. Natural Product Letters (1999), 14(1), 11-15, Mytilus edulis galloprovincialis, from two species of Eucalyptus. Biosci Biotechnol Biochem 60:1522-1523; Terada Y, Saito J, Kawai T, Singh IP, Etoh H 1999 Structure-activity relationship of phloroglucinol compounds from Eucalyptus, as marine antifoulants. Biosci Biotechnol Biochem 63:276-280), antibacterial activity (Yamakoshi Y, Murata M, Shimizu A, Homma S. Isolation and characterization of macrocarpals B-G antibacterial compounds from Eucalyptus macrocarpa. Bioscience, Biotechnology, and Biochemistry (1992), 56(10), 1570-6; Osawa K, Yasuda H, Morita H, Takeya K, ltokawa H Macrocarpals H, I, and J from the leaves of Eucalyptus globulus. Journal of Natural Products (1996), 59(9), 823-827), inhibitory activity of HIV-Rtase (Nishizawan M, Emura M, Kan Y, Yamada H, Ogawa K, Hamanaka N 1992 Macrocarpals HIV-reverse transcriptase inhibitors of Eucalyptus globulus. Tetrahedron Lett 33:2983-6), angiotensin-converting enzyme (Saeki T, Ohsawa K, Yasuda H Angiotensin-converting enzyme inhibitors and foods and beverages containing them. JP11060498A2), aldose reductase (Murata M, Yamakoshi Y, Homm S, Arai K, Nakamura Y. 1992 Macrocarpals, antibacterial compounds from Eucalyptus, inhibit aldose reductase. Biosci Biotechnol Biochem 56: 2062-3), tumour inhibition (Takasaki M, Konoshima T, Kozuka M, Tokuda H 1995 Anti-tumor-promoting activities of euglobals from Eucalyptus plants. Biol Pharm Bull 18:435-438), ceramide formation promoter (Kusuoku H, Shibuya Y, Ohashi S, Takagi Y, Okubo K. Macrocarpals and ceramide formation promoters containing them JP2001055325A2) and glucosyltransferase (Osawa K, Saeki T, Yasuda H, Morita H, Takeya K, Itokawa H 1998 Antibacterial activity of Eucalyptus globulus on cariogenic bacteria and its inhibitory effect on glucosyltransferase. Nat Med (Tokyo) 52:32-37). In addition, they play a major ecological role in Australian forests since they act as powerful antifeedants against insect and marsupial herbivores (Pass DM, Foley, WJ, Bowden B 1998 Vertebrate herbivory on Eucalyptus - identification of specific feeding deterrents for common ringtail possums (Pseudocheirus peregrinus) by bioassay-guided fractionation of Eucalyptus ovata foliage. J Chem Ecol 24:1513-1527, Lawler et al. 2000 see above).

Formylated phloroglucinol compounds probably do not occur in all eucalypts. Preliminary studies (Eschler BM, Pass DM, Willis R, Foley WJ (2000) Distribution of foliar formylated phloroglucinol derivatives amongst Eucalyptus species. Biochem Syst Ecol 28:813-824) suggest that FPCs are concentrated in the informal subgenus Symphyomyrtus. They seem to be absent from the informal subgenus Monocalyptus and from the monospecific informal subgenus Idiogenes (*E. cloeziana*).

Within our studies in evaluating plant extracts in order to find new activities related to mental diseases and disorders we prepared extracts from Eucalyptus sp. and tested these in a complex assay to demonstrate their ability to affect neurotransmitters levels.

Components from the oils of *Eucalyptus* have already been brought into connection with some positive psychological effects, see e.g. GB 2374284, DE 44 47 336 A1 and Göbel et al., Cephalalgia 14, 228-234 (1994).

It is a problem of the present invention to make available new compounds or compositions useful in the treatment (including therapy and prophylaxis) of various neurological and/or mental diseases or disorders or improving the neurological or mental situation also in essentially healthy persons.

Surprisingly, extracts obtained from *Eucalyptus globulus* leaves comprising certain compounds which belong to the class of acylphloroglucinol derivatives as well as some purified compounds from this class out of such extracts can be shown to exhibit an enhancing effect on the neurotransmitter concentration by unknown mechanism, as is described in more detail below.

Even more surprisingly, the compounds cannot be found in the oil from Eucalyptus which contains mainly terpenoids, and thus a new class of compounds is made available in the treatment of the neurological and/or mental condition of animals, especially humans.

The invention therefore relates, in a first embodiment, to a compound of the formula I or a mixture of compounds of the formula I, 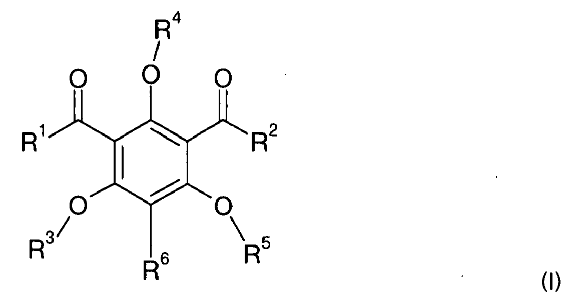 wherein
R' and R² are, independently of each other, hydrogen or linear or branched C₁-C₁₀-alkyl;
R³ and R⁴ are, independently of each other, hydrogen or linear or branched C₁-C₁₀-alkyl;
R⁵ is hydrogen or linear or branched C₁-C₁₀-alkyl;
and
R⁶ is linear or branched C₁-C₁₂-alkyl that is itself substituted with C₃-C₂₅-hydrocarbyl with at least one cyclic element that is saturated or includes one or more isolated - not conjugated or cumulated - double and/or triple carbon-carbon bonds and that is unsubstituted or substituted by one or more substituents independently selected from the group consisting of hydroxyl, linear or branched C₁-C₅-alkyl, linear or branched C₁-C₅-alkyliden, (linear or branched C₁-C₅-alkyl)oxy, hydroxyl-C₁-C₅-alkyl, oxo, linear or branched C₁-C₁₀alkyl substituted with oxo and cycloalkyl with 3 to 10 carbon atoms moieties, the cycloalkyl itself unsubstituted or substituted with one or more substituents selected from hydroxyl, linear or branched C₁-C₅-alkyl, linear or branched C₁-C₅-alkyliden, (linear or branched C₁-C₅-alkyl)oxy, hydroxyl-C₁-C₅-alkyl, oxo and linear or branched C₁-C₁₀alkyl substituted with oxo;
or one of R³ and R⁵ is hydrogen or linear or branched C₁-C₁₀-alkyl, the other is a bond to the C₃-C₂₅-hydrocarbyl part of the C₁-C₁₂-alkyl substituted with unsubstituted or substituted C₃-C₂₅-hydrocarbyl as defined above for R⁶ which thus, bound via a first bond to the O to which R³ or R⁵ is bound and via a second bond to the C₁-C₁₂-alkyl part of R⁶, forms a C₃-C₂₅-hydrocarbondiyl with at least one cyclic element that is saturated or includes one or more isolated - not conjugated or cumulated - double and/or triple carbon-carbon bonds, wherein one to three non-terminal CH₂ groups may be replaced by O, and that is unsubstituted or substituted by one or more substituents independently selected from the group consisting of hydroxyl, linear or branched C₁-C₅-alkyl, linear or branched C₁-C₅-alkyliden, (linear or branched C₁-C₅-alkyl)oxy, hydroxyl, hydroxyl-C₁-C₅-alkyl, oxo, linear or branched C₁-C₁₀alkyl substituted with oxo, and cycloalkyl with 3 to 10 carbon atoms moieties, the cycloalkyl itself unsubstituted or substituted with one or more substituents selected from hydroxyl, linear or branched C₁-C₅-alkyl, linear or branched C₁-C₅-alkyliden, (linear or branched C₁-C₅-alkyl)oxy, hydroxyl, hydroxyl-C₁-C₅-alkyl, oxo and linear or branched C₁-C₁₀alkyl substituted with oxo, which C₃-C₂₅-hydrocarbondiyl thus together with the O binding R³ or R⁵ in formula I, the carbon binding said O and the carbon binding R⁶ in formula I, the bond connecting these two carbons and the C₁-C₁₂-alkyl bound in the position of R⁶ forms a further ring,
with the proviso that the carbohydryl and the alkyl part of R⁶ together have at least 10 carbon,
and/or a tautomer or tautomers, a solvate or solvates, an ester or esters and/or a salt or salts thereof,
for use as central nervous system (CNS) activity modulators in the prophylactic and/or therapeutic treatment of an individual, especially desiring or requiring such treatment.

Other and especially preferred embodiments of the invention are given below and in the claims, which are incorporated herewith by reference into the description also as part thereof.

The general expressions, within the present disclosure, preferably have the following meaning, where in each embodiment one, more than one or all more general expressions may be replaced with the more specific definitions, thus forming preferred embodiments of the invention:

Alkyl can be linear or branched, that is, straight-chained or having one or more branchings. Preferred are methyl, ethyl, n-propyl, isopropyl, n-butyl, isobutyl, sec-butyl, tert-butyl, n-pentyl or isopentyl.

Hydrocarbyl R⁶ preferably consists of at least one cyclic element, especially up to three cycles, selected from the group consisting of simple carbocyclic moieties, e.g. C₃-C₁₂-cycloalkyl, condensed polycyclic moieties, such as condensed di-, tri- or tetracyclic moieties with 4 to 25 ring carbon atoms, bridged polycyclic moieties with 5 to 25 ring carbon atoms, such as bicyclo (preferably with one or more bridging carbon atoms to distinguish over bicyclo (dicyclic) systems with only a bond between the carbon atoms common to both rings (bridgeheads)), tricyclo, tetracyclo or pentacyclo moieties, and mono- or polyspiro-carbocyclic moieties, such as mono- or dispiro-carbocyclic moieties; or any combination of such cyclic moieties. It can be substituted by one or more, especially by up to five, of the substituents mentioned above.

Hydrocarbyl preferably has 4 to 20 carbon atoms (without counting carbons of substituents).

Non-terminal CH₂ mean any such groups that are bound to two carbon atoms.

Preferably, hydrocarbyl in R⁶ is selected from the group consisting of ring systems with the following formulae, the waved line marking the end of the bond at the preferred place of binding to the linear or branched C₁-C₁₂-alkyl (which is preferably methyl or ethyl, or is isopentyl substituted by hydrocarbyl at the C', that is, isopentan-1,1-diyl) substituted by said hydrocarbyl with which it forms R⁶:

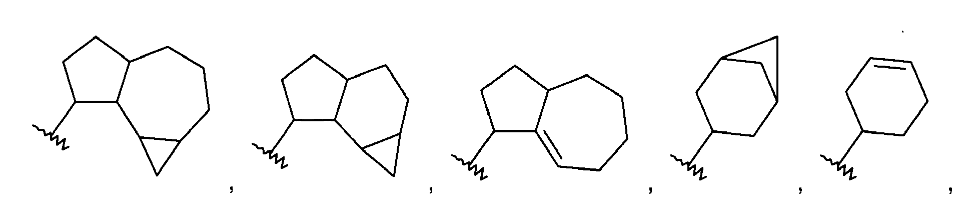

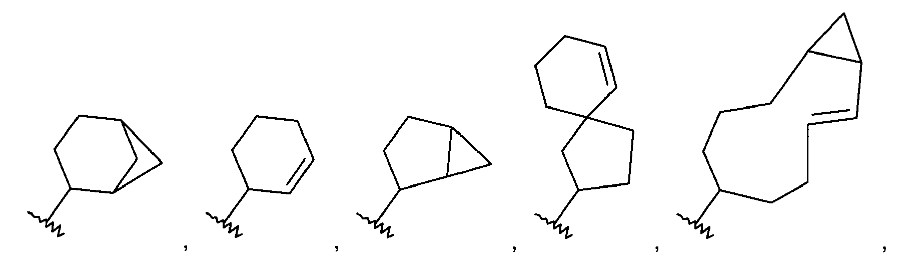

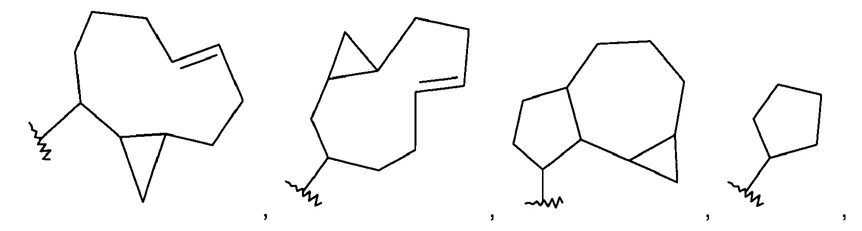

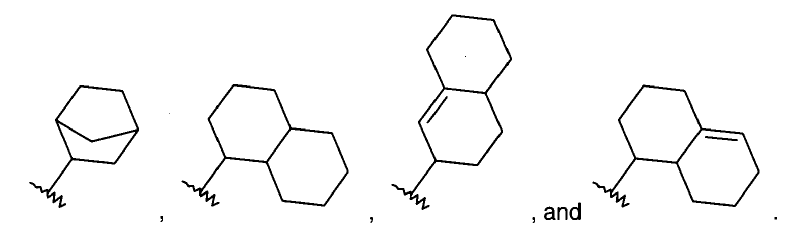

Isolated double bonds means that these double bonds are not conjugated (as in -CH=CH-CH=CH-) or cumulated (as in -CH=C=CH-), but at least separated by three carbon atoms bound to each other by single bonds. Preferably, up to three, more preferably 1 or two triple and/or (preferably) double bonds are present in hydrocarbyl.

Where R³ or R⁵ and R⁶ together form a C₃-C₂₅-hydrocarbondiyl, the latter is preferably a hydrocarbyl as defined for R⁶ bound on the one hand with one bond to the linear or branched C₁-C₁₀-alkyl moiety via which it is bound to form R⁶, on the other hand with the other bond to the oxygen binding R³ or R⁵ in formula I. More preferably, hydrocarbondiyl in R⁶ is selected from the group consisting of ring systems with the following formulae, the waved line marking the end of the bond of the preferred place of binding to the linear or branched C₁-C₁₂-alkyl (preferably defined as given above the preferred formulae for hydrocarbyl R⁶) and the asterisk marking the preferred end of the bond where the O that binds R³ or R⁵ in formula I is bound:

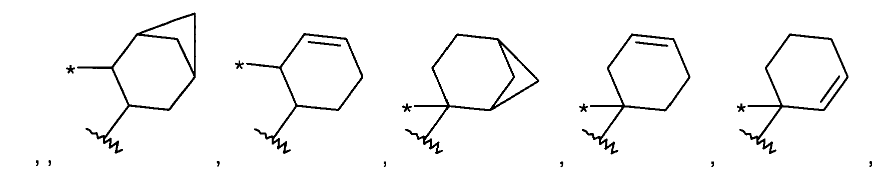

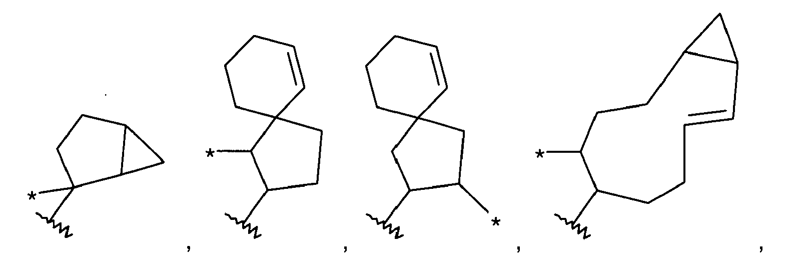

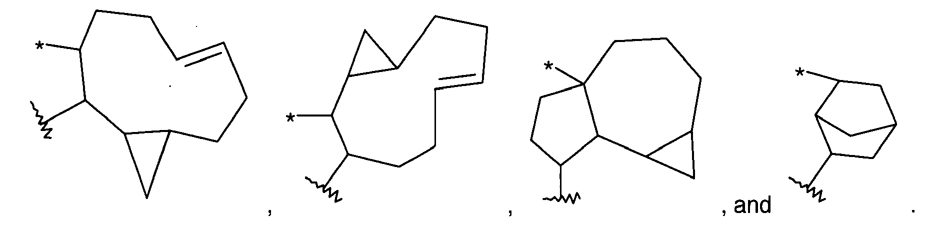

Any double bonds (also if shown in a preferred constitution in the formulae above for hydrocarbyl and hydrocarbondiyl) may be present in the cis- or trans- form or both, preferably in the form shown.

Very preferred compounds of the formula I useful according to the invention, each of which may be of used alone or in combination of two or more of them, are selected from the group represented by the following list (where preferably the compound(s) are characterized by either of their formula, their name and/or (especially) the compound falling under the CAS-number, these compounds here being incorporated by reference) given are meant:

| Structures according to Dictionary of Natural Products 2007 | Name | CAS-No. |
|---|---|---|
| 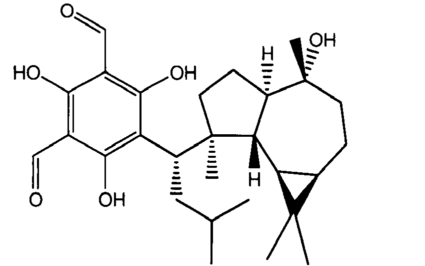 | Macrocarpal B | 142698-60-0 |
| 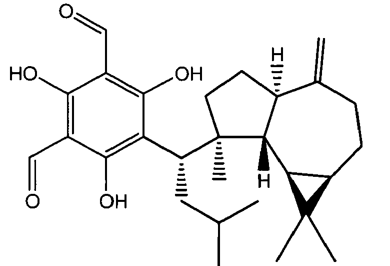 | Macrocarpal C | 142628-53-3 |
| 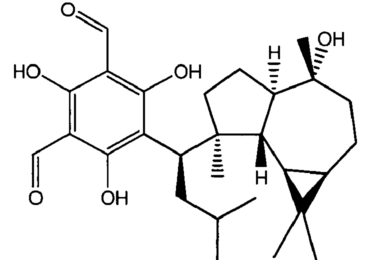 | Macrocarpal A | 132951-90-7 |
| 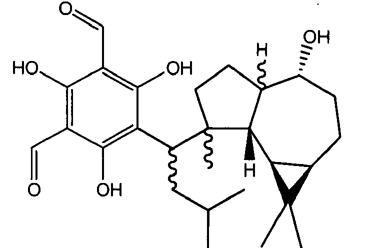 | Antibiotic GR 95647X | |
| 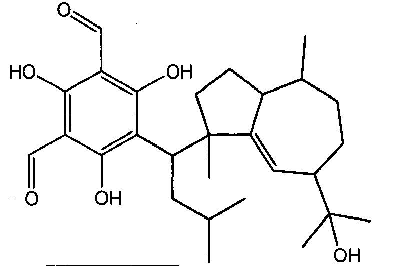 | Macrocarpal G | |
| 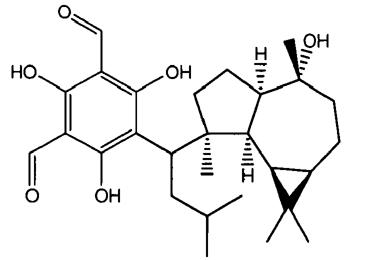 | Euvimal 1 | 144372-45-2 |
| 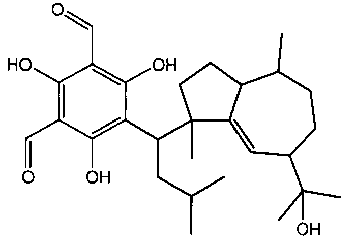 | Macrocarpal D | |
| 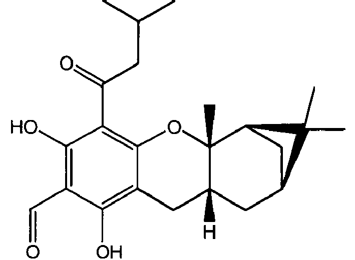 | Euglobal G1 | 130304-62-0 |
| 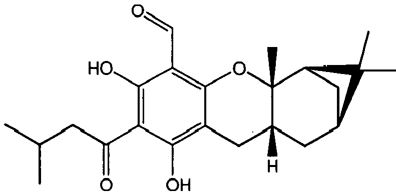 | Euglobal G2 | 130288-57-2 |
| 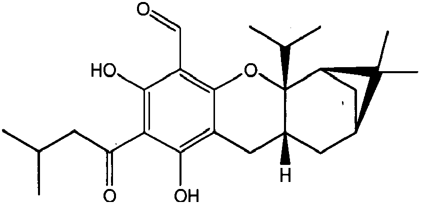 | Euglobal G8 | |
| 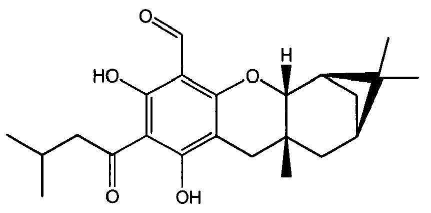 | Euglobal G9 | |
| 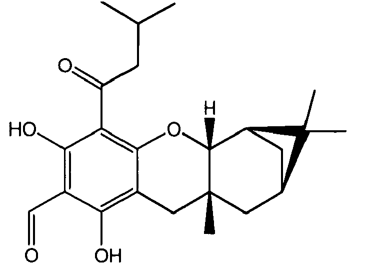 | Euglobal G10 | |
| 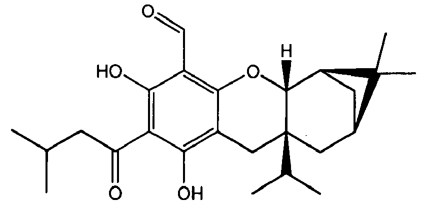 | Euglobal G11 | |
| 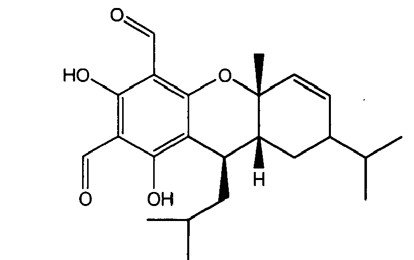 | Euglobal Ia₁ | 77844-93-0 |
| 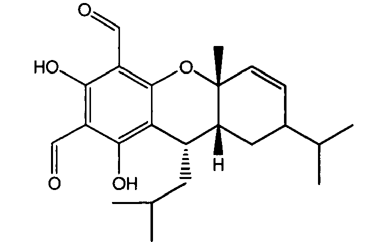 | Euglobal Ia₂ | 77794-63-9 |
| 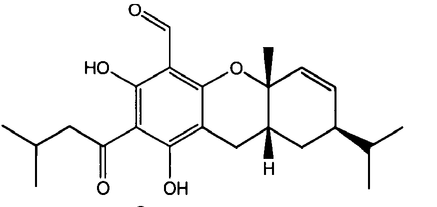 | Euglobal IIc | 77794-62-8 |
| 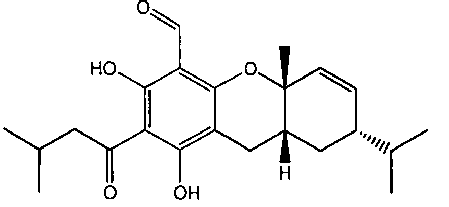 | Euglobal IIc Δ7-Isomer | 208182-90-5 |
| 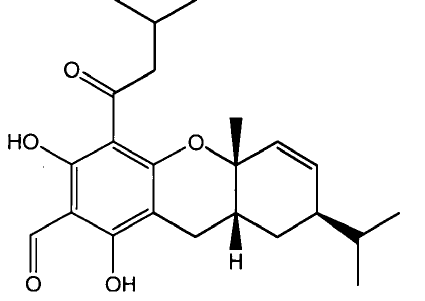 | Euglobal T1 | 137100-74-4 |
| 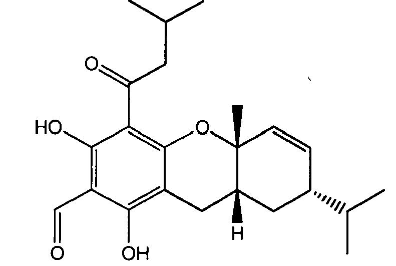 | Euglobal T1 Δ7-Isomer | 208182-91-6 |
| 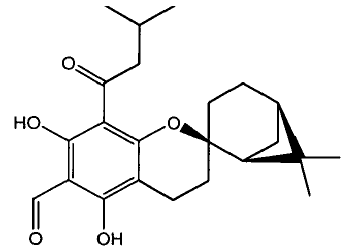 | Euglobal G4 | 163564-61-2 |
| 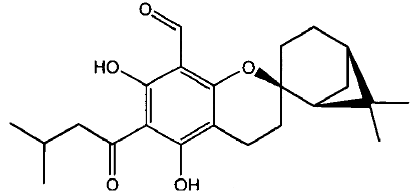 | Euglobal G3 | 130288-58-3 |
| 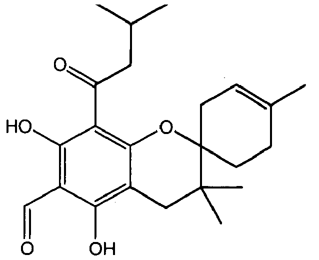 | Euglobal G12 | |
| 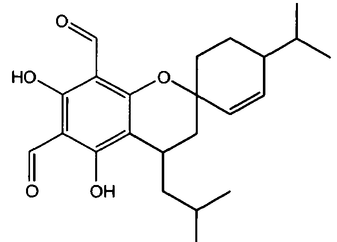 | Euglobal IIb | 77794-61-7 |
| 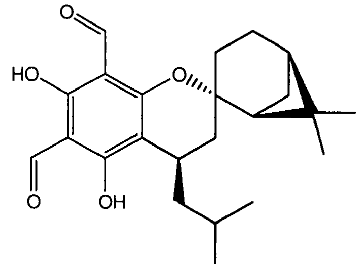 | Robustadial A, Euglobal Am 1 | 88130-99-8 |
| 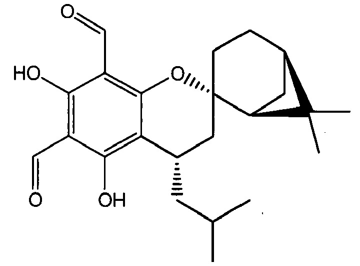 | Robustadial A 7-Epimer, Euglobal Am 2 | 88197-30-2 |
| 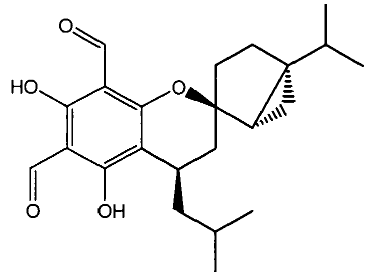 | Euglobal Ic | 77794-60-6 |
| 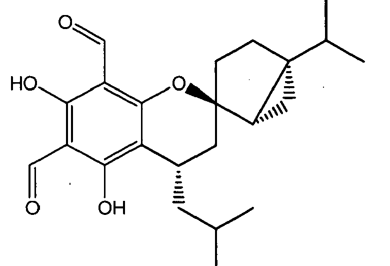 | Euglobal IIa | 77844-92-9 |
| 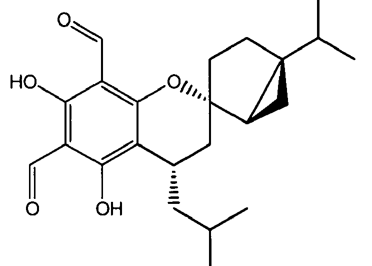 | Euglobal Bl 1 | 163437-53-4 |
| 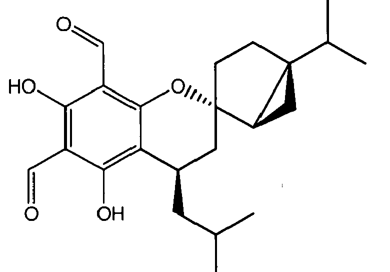 | Euglobal Ib | 77844-94-1 |
| 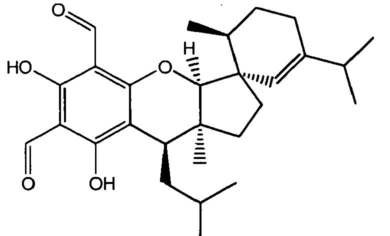 | Euglobal In 1 | 168706-12-5 |
| 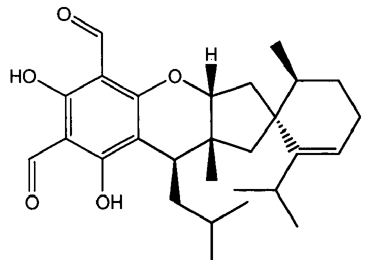 | Euglobal In 2 | 173357-28-3 |
| 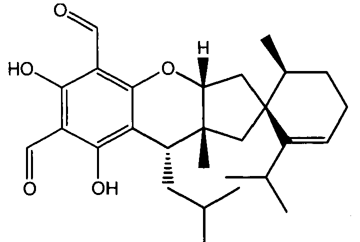 | Euglobal In 3 | 173401-70-2 |
| 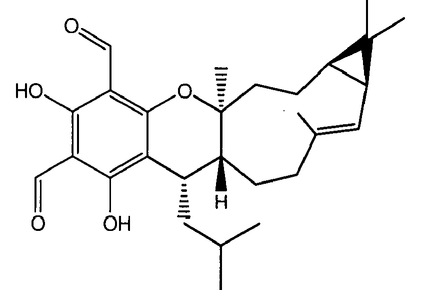 | Euglobal III | 76449-26-8 |
| 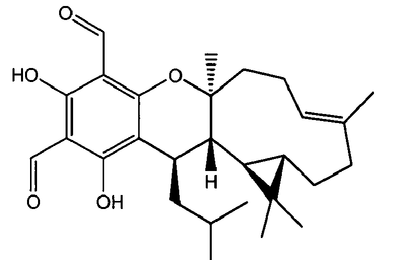 | Euglobal IVb | 82864-79-7 |
| 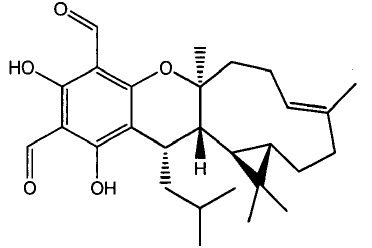 | Euglobal IVa | 77794-65-1 |
| 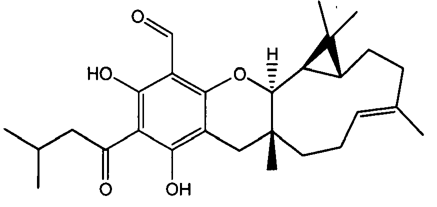 | Euglobal VII | 77794-64-0 |
| 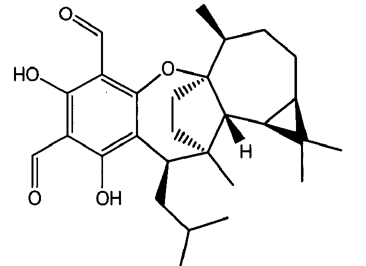 | Euglobal V | 77809-89-3 |
| 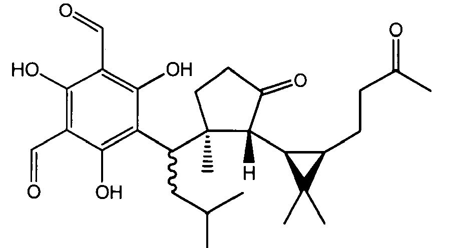 | Eucalyptone | 172617-99-1 |
| 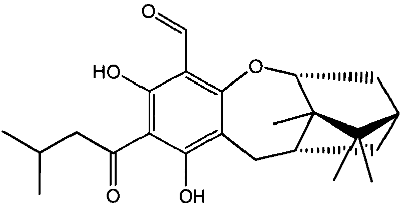 | Euglobal G5 | 163564-62-3 |
| 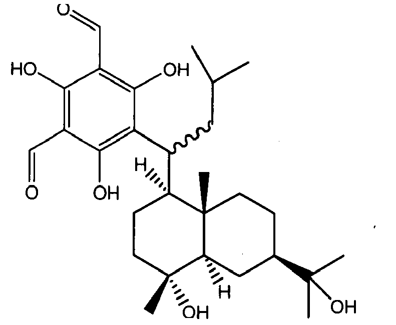 | Macrocarpal I | 179388-54-6 |
| 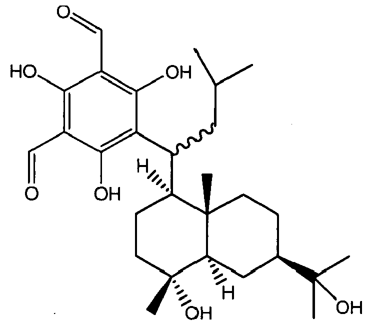 | Macrocarpal J, Epimer of Macrocarpal I | 179603-47-5 |
| 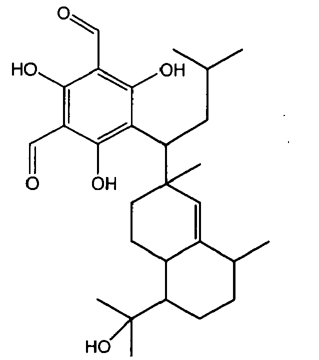 | Macrocarpal D | 142628-54-4 |
| 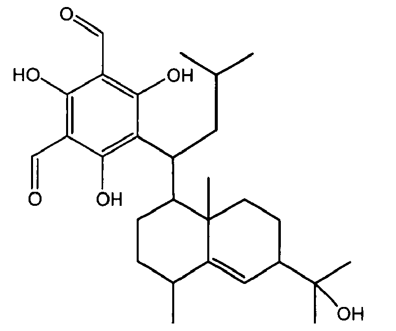 | Macrocarpal E | |
| 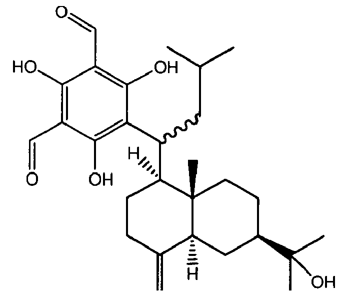 | Macrocarpal H | 179388-53-5 |
| 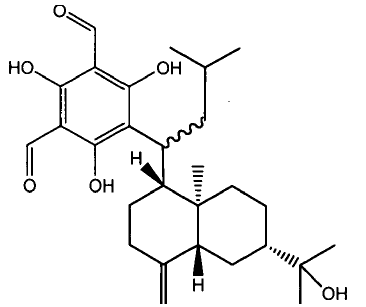 | Macrocarpal K | 218290-59-6 |

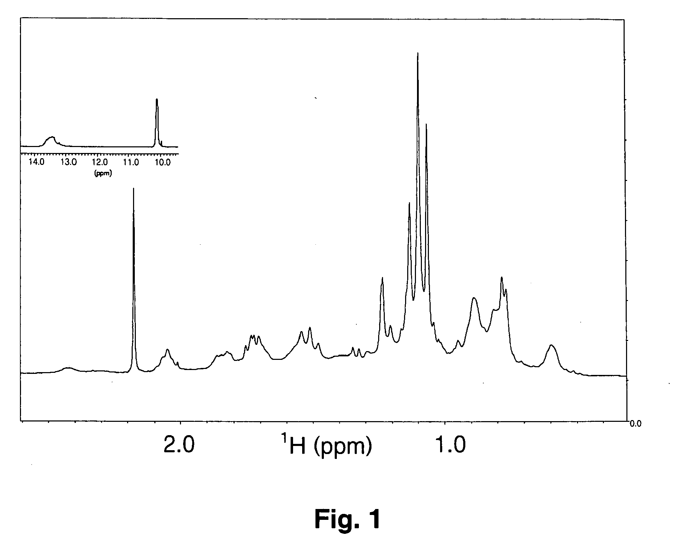

and a compound named "unknown FPC" herein that is characterized by an ¹H-NMR spectrum as shown in Fig. 1 and/or by the isolation method described in Example 2.

Highly preferred is the use of a compound named Eucalyptone, and/or a compound of the formula I called "unknown FPC" herein (which, in free form, as tautomer or tautomers, as solvate or solvates, as ester or esters and/or as salt or salts is also en embodiment of the invention as such, which is characterized by an ¹H-NMR spectrum as given in Fig. 1, and/or by an isolation method as described in Example 2, or a salt thereof, or mixtures of these two compounds and/or their salts, or the use of an extract comprising compounds of the formula I, preferably obtainable as described below, preferentially where the compounds of the formula I represent 20 to 100 % of the dry weight of the extract, more preferably 50 to 100 %.

Where ever within this disclosure a compound of the formula I, or a mixture of compounds of the formula I, is mentioned, this is intended to include the free (enriched or essentially pure) form and/or one or more (especially pharmaceutically acceptable) salts (where salt-forming groups, such as phenolic OH-groups, are present), solvates, ester and or tautomers (where tautomerism, e.g. of the oxo/enol type, is possible), or mixtures of two or more of those specific forms. A mixture may be the result of an extraction and/or of admixing two or more compounds of the formula I.
Tautomers present can (without excluding other possible tautomers or tautomer mixtures) e.g. be represented by the following tautomeric forms of the central part of formula I:

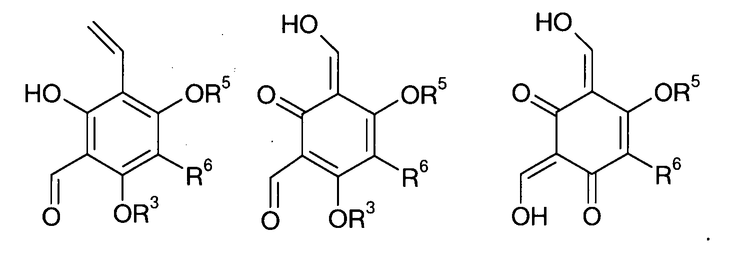

Solvates are e.g. hydrates or solvates with other solvents, e.g. used during extraction or working-up.

Esters can be esters of one or more (preferably) organic acids (such as carboxylic or sulfonic acid, e.g. C₁-C₁₀-alkanoyl or) and/or one or more inorganic acids (e.g. a halohalic acids such as HCl or the like, then leading to the corresponding halo, such as fluoro, chloro, bromo or iodo compound) of a compound of the formula I carrying one or more hydroxyl groups which is esterified at one or more of these hydroxyl groups.

The term "salt(s)", as employed herein, denotes salts formed with inorganic and/or organic acids and bases. Pharmaceutically or nutraceutically acceptable (i.e., non-toxic, physiologically acceptable) salts are preferred, although other salts are also useful, e.g., in isolation or purification steps which may be employed during preparation. Salts of a compound of the formula I may be formed, for example, by reacting a acylphloroglucinol derivative of the invention with an amount of base, such as an equivalent amount, in a medium such as one in which the salt precipitates or in an aqueous medium followed by lyophilization, or by any other customary method including ion exchange chromatography or batch reaction with ion exchangers.

For example, the acylphloroglucinol derivatives of the present invention which contain an acidic moiety (especially a phenolic OH group) may form salts with a variety of organic and inorganic bases. Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, N-methyl-D-glucamines, N-methyl-D-glucamides, t-butyl amines, and salts with amino acids such as arginine, lysine and the like. Also mixed salts (with more than one counterion, or where the counterions have different groups (e.g. amino groups and carboxylic groups) are included.

Further, the compounds of formula I may be present as mixtures of or as pure stereoisomers, so that these are comprised under formula I, such as those which may exist due to asymmetric carbons on the various substituents, including enantiomeric forms (which may exist even in the absence of asymmetric carbons) and diastereomeric forms. Individual stereoisomers of the acylphloroglucinol derivatives of the present invention may, for example, be substantially free of other isomers, or may be admixed, for example, as racemates or with all other, or other selected, stereoisomers.

"Comprising" or "including" or "having" where used herein is meant not to be limiting to any elements stated subsequently to such term but rather to encompass one or more further elements not specifically mentioned with or without functional importance, that is, the listed steps, elements or options need not be exhaustive. In contrast, "containing" would be used where the elements are limited to those specifically after "containing".

Where "about" or "essentially" is used, this preferably means that a given numerical value may deviate to a certain extent from the value given after "bout", e.g. preferably by ± 20 % of the given numerical value, more preferably by ± 10 %.

"Obtainable" means that a product (e.g. compound) may be obtained, preferably that it is obtained by the specified method.

Where ratios of components are given in %, this means weight-%, if not indicated otherwise.

By the term CNS activity modulators, it is meant especially that the compounds or mixtures according to the invention can be used for treating (therapeutically of prophylactically) either in patients or in healthy persons. Use as CNS activity modulators thus especially means use for the modulation, especially stimulation of CNS activity.

The compounds of the invention may be shown to increase attention in a test of attention for rodents (Robbins, J. Neuropsychiatry Clin. Neurosci. (2001) 13, 326-35), namely the 5-choice serial reaction time test (5-CSRTT). In this test, the rat must observe a wall containing 5 holes. When a light flash appears in one of them, the rat must respond with a nose-poke into the correct hole within 5 sec. in order to receive a food pellet reward, delivered to a feeder in the opposite wall.

Compounds of the invention may also show learning/memory enhancing effects in the social recognition test in mice and rats (Ennaceur and Delacour, Behav. Brain Res. (1988) 31, 47-59).

Most importantly, however, the compounds of the present invention can be shown to influence the transport process of neurotransmitters in rat synaptosomes. These are vesicles formed by isolated nerve endings in extracorporeal preparations (Gray EG, Whittaker VP (1962) The isolation of nerve endings from brain: an electronmicroscopic study of cell fragments derived by homogenization and centrifugation. J. Anat. 96:79-88). The test system is constructed in order to obtain variations in the transport in a full cell system. As consequence the results will be quantified on the level of specific neurotransmitter binding but not of the mode of action.

Compounds of the formula I, or mixtures of compounds of the formula I, in view of the activities shown especially in the Examples, are useful in modulating several brain functions.

Due to their pharmacological profiles e.g. shown in the Examples, compounds of the formula I are anticipated to be useful for the treatment of diseases or conditions as diverse as CNS related diseases (preferred), also peripheral nervous (PNS) related diseases especially insofar as they also affect or are affected by CNS activity, e.g. diseases related to inflammation, pain and withdrawal symptoms caused by an abuse of chemical substances; diseases or disorders related to the CNS include general anxiety disorders, cognitive disorders, learning and memory deficits and dysfunctions, Alzheimer's disease, ADHD, Parkinson's disease, Huntington's disease, ALS, prionic neurodegenerative disorders such as Creutzfeld-Jacob disease and kuru disease, Gilles de la Tourette's syndrome, psychosis, depression and depressive disorders, mania, manic depression, schizophrenia, the cognitive deficits in schizophrenia, obsessive compulsive disorders, panic disorders, eating disorders, narcolepsy, nociception, AIDS-dementia, senile dementia, mild cognitive dysfunctions related to age, autism, dyslexia, tardive dyskinesia, epilepsy, and convulsive disorders, post-traumatic stress disorders, transient anoxia, pseudodementia, pre-menstrual syndrome, late luteal phase syndrome, chronic fatigue syndrome and jet lag. Furthermore, compounds of the invention may be useful for the treatment of endocrine disorders, such as thyrotoxicosis, pheochromocytoma, hypertension and arrhythmias as well as angina pectoris, hyperkinesia, premature ejaculation and erectile difficulty. Still further, compounds of the invention may be useful in the treatment of inflammatory disorders (Wang et al., Nature 2003, 421,384), disorders or conditions including inflammatory skin disorders, Crohn's diesease, inflammatory bowel disease, ulcerative colitis and diarrhoea. Compounds of the invention may further be useful for the treatment of withdrawal symptoms caused by termination of the use of addictive substances, like tobacco, nicotine, opioids, benzodiazepines and alcohol. Also, compounds of the invention may be useful for the treatment of pain, e.g. caused by migraine, postoperative pain, phantom limb pain or pain associated with cancer. The pain may comprise inflammatory or neuropathic pain, central pain, chronic headache, pain related to diabetic neuropathy, to post therapeutic neuralgia or to peripheral nerve injury.

The compounds useful according to the invention can for example preferentially be used in the treatment and/or prophylaxis of depression, depressive disorders, anxiety and affective disorders, e.g. especially where one or more of the following symptoms or disorders is present and/or to be avoided prophylactically: general anxiety disorders, fear, cognitive disorders, learning and memory deficits and dysfunctions, manic depression, obsessive compulsive disorders, panic disorders, eating disorders, narcolepsy, sleeping disorders, such as chronic fatigue syndrome and jet lag, lack of ability to make decisions, lack of concentration, inferiority feeling, lethargy, anergy, lack of drive, dysphoria, melancholy, diminished emotional well-being, mood depresssion, low spirits, inner restlessness, feelings of being guilty, delusion of being guilty, stiff thinking, inhibition of thinking, circular way of thinking, feeling of helplessness, feeling of hopelessness, lack of appetite, enlarged susceptibility to infection due to a depressed mood, decreased cognitive abilities and/or other symptoms, as well as for mood-lifting in healthy persons, and/or also in the treatment of schizophrenia or psychotic diseases.

Especially, the term CNS activity modulators includes that the compounds useful according to the present invention show a mood lifting activity, increase cognitive performance, positively affect acquisition of memory, enhance working memory, positively affect motivation, positively influence behavioural initiative and thus increase general quality of living, and thus can be used to avoid or ameliorate any lack of these conditions.

These activities are believed to be mainly due to effects that are manifested in brain, such as the elevation of the level of one or more neurotransmitters, such as serotonin (e.g. by inhibition of its re-uptake or other mechanisms such as biosynthesis or release from storage sites), dopamine, norepinephrine or the like, and/or the inhibition of certain neurotransmitter receptors, such as cholinergic, α-adrenergic or histamine receptors, though other mechanisms of action shall not be excluded here.

A mixture of compounds of the formula I can, for example, be a mixture that is formed by extraction of a plant material, and/or by mixing two or more enriched or isolated compounds of the formula I or one or more compounds of the formula I to an extract or the like. Preferably, the compound is or the compounds are in enriched or isolated form, compared with their natural occurrence.

The efficiency of the compounds as CNS activity modulators or compound mixtures useful according to the invention can thus be shown by *in vitro* or especially in cellular or cell based assays as shown above or in the Examples.

In addition, the efficiency can be demonstrated *in vivo,* e.g. in animal assays or in clinical studies.

As an example of animal assays, the "Forced Swimming Test" with rats may be mentioned. During this assay rats are brought, for a defined period of time of 5 minutes, into a situation that is hopeless for them (glass cylinder filled with water). The rats react in this test with a temporary rigor called immobilisation period which is interpreted as the correlate of depression. If rats are treated with an anti-depressive substance before this test, the immobilisation period is diminished. A comparison is possible with known antidepressives, such as the tricyclic antidepressant imipramine. In order to be effective, usually the test substances need to be administered for a period of several days.

The compounds of the invention are also commercially useful as research chemicals for establishing animal models and the like for the diseases or disorders or activities mentioned.

The compound of the formula I, or a mixture of compounds of the formula I, may be used according to the invention e.g. in the form of nutraceutical or as a pharmaceutical composition, or they may be added as such e.g. to food, cosmetic products (such as lipsticks, make-up, ointments, oils, gels or the like) or other consumables.

A "nutraceutical" (sometimes also called "Functional Food", "Functional Food products", "Foodsceuticals", "Medicinal Food" or "Designer Food") according to the present invention is defined as food product (including beverages) suitable for human consumption - the expression comprises any fresh or processed food having a health-promoting and/or disease-preventing property beyond the basic nutritional function of supplying nutrients, including food made from functional food ingredients or fortified with health-promoting additives, especially with an effects in the prophylaxis or treatment of one or more of the disorders mentioned herein, especially allowing for a mood lifting function and in which a compound of the formula I or a mixture of compounds of the formula I according to the invention is added as an ingredient (especially additive) as health benefit agent, especially in an effective amount, as well as any partially or totally artificially composed food.

The nutraceuticals may be manufactured according to any suitable process, preferably comprising
(a) extraction of one or more compounds and/or mixture of compounds from one or more of the biological materials mentioned above and below, especially according to a method as described below; and
(b) adding the resulting one or more compounds and/or mixtures of compounds as ingredient in the preparation of the functional food product.

This manufacture therefore also constitutes an embodiment of the invention.

Further processing steps may follow, such as drying, freeze-drying, pasteurizing, sterilizing, freezing, dissolving, dispersing, filtering, centrifuging, confectioning, and the like.

When one or more compounds and/or a compound mixture according to the invention are added to a food product, this results in a functional food product according to the invention.

Preferably, the food product comprises 0,001 to 50 %, e.g. 0,001 to 3 % by weight of compounds of the formula I.

Further additives may be included, such as vitamins, minerals, e.g. in the form of mineral salts, unsaturated fatty acids or oils or fats comprising them, other extracts, or the like.

The functional food products according to the invention may be of any food type. They may comprise one or more common food ingredients in addition to the food product, such as flavours, sugars, fruit, minerals, vitamins, stabilisers, thickeners, dietary fibers, protein, amino acids or the like in appropriate amounts, or mixtures of two or more thereof, in accordance with the desired type of food product.

Examples of basic food products and thus of functional food products according to the inventtion are fruit or juice products, concentrates thereof, lemonades; extracts, e.g. coffee, tea, green tea; dairy type products, frozen confectionary products, baked goods, spreads, e.g. margarine, butter, peanut butter honey; snacks, pasta products or other cereal products, ready-to-serve-dishes; frozen food, tinned food, syrups, sauces, fillings, dips, chewing gums, sherbet, spices, cooking salt, instant drink powder or other instant powders e.g. for pudding or other desserts; or the like.

Flavour or other additives, such as one or more selected from stabilizers, e.g. thickeners; colouring agents, such as edible pigments or food dyes; bulking agents, polyols, such as xylitol, mannitol, maltitol or the like; preservatives, such as sodium or potassium benzoate, sodium or calcium carbonate or other food grade preservatives; antioxidants, such as ascorbic acid, carotionoids, tocopherols or polyphenols; mono-, oligo- or polysaccharides, such as glucose, fructose, sucrose, soy-oligosaccharides, xylo-oligosaccharides, galacto-oligosacharides; other artificial or natural non- or low-caloric sweeteners, such as aspartame or acesulfame; acidifiers in the form of edible acids, such as citric acids, acetic acid, lactic acid, adipic acid; flavours, e.g. artificial or natural (e.g. botanical flavours); emulsifiers; diluents, e.g. maltodextrose; wetting agents, e.g. glycerol; stabilizers; coatings; isotonic agents; absorption promoting or delaying agents; or the like may also be present.

The nutraceutical compositions according to the present invention can alternatively be prepared in various forms, such as granules, tablets, pills, suppositories, capsules, suspensions, salves, lotions, suspensions, and the like.

The one or more compounds or compound mixture according to the invention can also be comprised in confectioned formulations (also including the pure compounds or compound mixtures) to be added to foods including beverages, e.g. in the form of powders or granules, e.g. freeze-dried or spray-dried, concentrates, solutions, dispersions or other instant form, or the like.

For use in a pharmaceutical composition (which can be used e.g. as a drug or as a food supplement, comparable to vitamin formulations), a compound of the formula I and/or a pharmaceutically acceptable salt thereof may be administered as single active agent or in combination with one or more other active agents of the formula I and/or a pharmaceutically acceptable salt thereof or especially other active agents commonly employed especially for the treatment of the disorders mentioned herein or further other disorders, e.g. one or more phytotherapeutics and/or common (e.g. chemical or biotechnological) pharmaceuticals, in any customary manner, e.g. enterally, such as rectally, e.g. in the form of suppositories, orally, for example in the form of tablets, capsules (e.g. hard or soft gelatine capsules), pills, sachets, powders, granules, or the like, or as nasal formulation, e.g. nasal drops, sprays or ointments, or as formulation for external (e.g. topic) administration, such as solutions, suspensions, ointment, lotions, creams, hydrogels, lipogels, micronized powders, sprays, aerosols, plasters (e.g. transdermal therapeutic systems) or the like, or parenterally, for example in the form of injection or infusion solutions or dispersions (including emulsions or suspensions), e.g. for i.v., i.m., s.c. or other administration.

For topical administration to the skin or mucous membranes the aforementioned compounds can e.g. be prepared as ointments, tinctures, creams, gels, solution, lotions; nasal sprays; aerosols and dry powder for inhalation; suspensions, shampoos, hair soaps, perfumes and the like. In fact, any conventional composition can be utilized in this invention. These preparations preferably comprise 0.1 to 50.0 percent by weight, preferably 0.3 to 20.0 percent by weight, of the active compound(s), based on the total weight of the composition.

For the treatment of the above or other disorders, the appropriate dosage of a compound (active ingredient) of the formula I or a mixture comprising such a compound will, of course, vary depending upon, for example, the host, the mode of administration and the nature and severity of the condition being treated as well as the relative potency of the particular agent of the invention employed. For example, the amount of active agent required may be determined on the basis of known in vitro and in vivo techniques, determining how long a particular active agent concentration in the blood plasma remains at an acceptable level for a therapeutic effect. In general, satisfactory results in animals are indicated to be obtained at daily dosages of from about 0.1 to about 1 000.00 mg/kg. For example, in humans, a possible indicated daily dosage is in the range of from about 1 to about 8000 mg/day p.o., (70 kg person), conveniently administered once or in divided doses up to 4 x per day or in sustained release form. Dosage forms accordingly suitably comprise from about 0.1 or 1.0 to about 500 or 2000 mg of a compound or compound mixture of the invention admixed with an appropriate pharmaceutically acceptable diluent or carrier therefore.

Pharmaceutical compositions contain, for example, from about 0.1 % to about 100 (e.g. about 99.9) %, preferably from about 20 % to about 60 %, of the active ingredient(s).

Examples for liquid compositions comprising a compound or compound mixture of the invention include, for example, a solid dispersion, an aqueous solution, e.g. containing a solubilising agent, a microemulsion and a suspension of a compound of formula I or a mixture of compounds of the formula I in the range of from 0.1 to 1 %, e.g. 0.5 %. The composition may be buffered to a pH in the range of, e.g. from 3.5 to 9.5, e.g. to pH 4.5, by a suitable buffer.

In the case of a combination, the pharmaceutical compositions for separate administration of the combination partners and/or those for administration in a fixed combination, i.e. a single galenical composition comprising at least two combination partners, according to the inventtion can be prepared in a manner known per se and are those suitable for enteral, such as oral or rectal, external and/or parenteral administration to mammals, including man, comprising a therapeutically effective amount of at least one pharmacologically active combination partner alone or in combination with one or more pharmaceutically acceptable carriers, especially suitable for enteral or parenteral application.

Pharmaceutical preparations for the combination therapy for enteral or parenteral or external administration are, for example, those in unit dosage forms, such as sugar-coated tablets, tablets, capsules, suppositories, ampoules, creams, lotions, plasters or ointments. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can instead with a single dosage unit also be reached by administration of a two or more dosage units.

In particular, a therapeutically effective amount of each of the combination partners may be administered simultaneously or sequentially and in any order, and the components may be administered separately (e.g. sequentially after fixed or variable periods of time), or as a fixed combination. For example, the method of treatment (including mitigation) of a disorder according to the invention may comprise (i) administration of the combination partner (a) (a compound of the present invention) in free or pharmaceutically acceptable salt form and (ii) administration of a combination partner (b) (e.g. a different compound of the present inventtion or an active ingredient of a different formula) in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners can be administered separately at different times during the course of therapy or concurrently in divided or single combination forms. Furthermore, the term "administering" also encompasses the use of a prodrug of a combination partner that convert *in vivo* to the combination partner as such. The instant invention is therefore to be understood as embracing all such regimes of simultaneous and/ or alternating treatment and the term "administering" is to be interpreted accordingly.

The effective dosage of the combination partners employed may vary, for example depending on the particular compound or pharmaceutical composition employed, the mode of administration, the disorder being treated, and/or the severity of the disorder being treated. Thus, the dosage regimen is selected in accordance with a variety of factors including the route of administration, metabolism by and the renal and hepatic function of the patient. A physician, clinician or veterinarian of ordinary skill can readily determine and prescribe the effective amount of the single active ingredients required to prevent, mitigate, counter or arrest the disorder. Optimal precision in achieving concentration of the active ingredients within the range that yields efficacy without toxicity requires a regimen based on the kinetics of the active ingredients' availability to target sites.

In accordance with the foregoing, the present invention also comprises the following embodiments which can be inserted wherever a compound or compound mixture "for use" is mentioned:
(1) A compound of the formula I, or a mixture of compounds of the formula I, for use in the diagnostic or especially therapeutic (including prophylactic) treatment of an animal, preferably a mammal, especially a human; especially for use as CNS activity modulator, more especially as a mood lifting agent, for example for use in the treatment (including mitigation) of any one or more disorders, especially of any one or more of the particular disorders set forth hereinbefore and hereinafter.
(2) A pharmaceutical or nutraceutical composition comprising a compound of the formula I, or a mixture of compounds of the formula I, as active ingredient together with a pharmaceutically acceptable diluent or carrier, especially for use in the therapeutic and/or prophylactic treatment of one or more of the diseases or disorders mentioned above.
(2') A pharmaceutical composition for the treatment or prevention of a disorder wherein CNS activity is dampened, more especially the level of neurotransmitters is too low in the brain or very especially in synaptic clefts, comprising a compound of the formula I, or a mixture of compounds of the formula I, and a pharmaceutically acceptable diluent or carrier.
(3) A method for the treatment of a disorder, especially any one or more of the particular disorders set forth hereinbefore, in a subject in need of such treatment, comprising administering a pharmaceutically effective amount of a compound of the formula I, a mixture of compounds of the formula I, especially to an individual in need thereof.
(3') A method for treating or preventing a disorder wherein CNS activity is dampened, more especially the level of neurotransmitters is too low in the brain or very especially in synaptic clefts, comprising administering to a mammal in need thereof a therapeutically effective amount of a compound of the formula I, or a mixture of compounds of the formula I, especially to an individual in need thereof.
(4) The use of a compound of the formula I, or a mixture of compounds of the formula I, for the manufacture of a medicament or food supplement for the treatment or prevention of a disease or disorder as mentioned above;
(4') The use of a compound of the formula I, or a mixture of compounds of the formula I, for the manufacture of a medicament or food supplement for a disease or disorder wherein CNS activity is dampened, more especially the level of neurotransmitters is too low in the brain or very especially in synaptic clefts, especially one or more of the disorders mentioned above.
(5) A method as defined above comprising co-administration, e.g. concomitantly or in sequence, of a therapeutically effective amount of compound of the formula I, or a mixture of compounds of the formula I, and a different pharmaceutically active compound and/or a pharmaceutically acceptable salt thereof, said different pharmaceutically active compound and/or salt thereof being especially for use in the treatment of any one or more of the disorders set forth hereinbefore or hereinafter.
(6) A combination product comprising a therapeutically effective amount of a compound of the formula I, or a mixture of compounds of the formula I, and a different pharmaceutically active compound and/or a pharmaceutically acceptable salt thereof, said second pharmaceutically active compound being especially for use or of use in the treatment of any one or more of the particular disorders set forth hereinbefore.

By "administering" herein is especially meant administration of a therapeutically effective dose of a compound of the formula I, or a mixture of compounds of the formula I, to a cell either in cell culture or especially to a patient. By "therapeutically effective dose" herein is preferably meant a dose that produces the effects for which it is administered.

The pharmaceutical or nutraceutical preparations may be sterilized and/or may contain adjuvants such as preservatives, stabilizers, binders, disintegrants, wetting agents, skin or mucuous membrane penetration enhancers, emulsifiers, salts for varying the osmotic pressure and/or buffers, or other ingredients, excipients or carrier materials known in the art.

Pharmaceutical grade or food grade organic or inorganic carriers and/or diluents suitable for oral and topical use can be used to formulate compositions containing the therapeutically-active compounds of the formula I. Diluents known in the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents. The pharmaceutical or nutraceutical compositions may also include one or more of the following: carrier proteins such as serum albumin; buffers; fillers such as microcrystalline cellulose, lactose, corn and other starches; binding agents; sweeteners and other flavoring agents; colouring agents; and polyethylene glycol. Those additives are well known in the art, and are used in a variety of formulations.

A "patient", "individual" or "subject" for the purposes of the present invention includes both humans and other animals. Thus, a compound of the formula I, or a mixture of compounds of the formula I, are applicable to both humans and animals. In the preferred embodiment the patient is a human. The patients will be treated either in prophylactic or therapeutic intention. However, also "healthy" persons may be treated, e.g. for generally lifting their mood or showing other of the preferred effects given above.

Generally, in the pharmaceuticals and/or the nutraceuticals the share of the compound of the formula I, or a mixture of compounds of the formula I, may be in the range from 0,001 to 100 % by weight, more preferably in the range from 0,01 to 30 % by weight.

### Method of manufacture

The compound of the formula I or compounds of the formula I (also called acylphloroglucinols in the following) can be prepared according to known chemical reactions or can be isolated from biological materials, e.g. from the organisms mentioned below. It is also possible to isolate precursors of the acylphloroglucinol derivatives and to prepare derivatives thereof according to known chemical reactions. The acylphloroglucinol derivatives can e.g. be isolated as described in the appended examples. The method for detection comprises high pressure liquid chromatography (HPLC) on reversed phase silica gel (C18) with water/ acetonitrilgradient as an elution solvent with UV (ultraviolet) as well as MS (mass spectroscopy) detection which are used for the product analysis and production optimization. It will be clear to those having ordinary skill in this art that the acylphloroglucinol derivatives of the present invention can be synthesized according to standard methods which for example can be deduced from the following publication: March's Advanced Organic Chemistry: Reaction, Mechanisms and Structure, 5th ed. by Michael B. Smith, Jerry March, Wiley-Interscience; 2001; Classics in Total Synthesis: Targets, Strategies, Methods by K. C. Nicolaou, E. J. Sorensen John Wiley & Son Ltd, 1996 and The Art and Science of Total Synthesis at the Dawn of the Twenty-First Century. Nicolaou KC Vour-loumis, D, Winssinger N, Baran PS., Angew Chem Int Ed Engl 2000, 39 (1): 44 -122.

Preferably, however, they or their precursors that may subsequently be used to form them are enriched or preferably isolated from biological materials, especially from plants of the genus *Eucalyptus* and some Microorganisms like *Pseudomonas aurantiaca* and *P. fluorescens, more especially from:*
*E. aggregata, E. albens, E. alpina, E. amplifolia, E. amygdalina, E. angustissima, E. apodophylla, E. approximans, E. archeri, E. argophloia, E. australiana, E. barberi, E. blakelyi, E. botryoides, E. brookeriana, E. camaldulensis, E. cinerea, E. cladocalyx, E. clarksoniana, E. citriodora, E. cloeziana, E. cneorifolia, E. coccifera, E. coolabah, E. cordata, E. coronata, E. dalrympleana, E. debeuzevillei, E. diversicolor, E. dawsonii, E. dorrigoensis, E. dumosa, E. dundasii, E. famelica, E. ficifolia, E. glaucescens, E. globulus, E. grandis, E. gregsoniana, E. gunnii, E. horistes, E. incrassata, E. jensenii, E. johnstonii, E. kartzoffiana, E. kochii, E. kybeanensis, E. largiflorens, E. leptopoda, E. leucoxylon, E. loxophleba, E. macarthurii, E. macrocarpa, E. maculata, E. mannifera, E. melliodora, E. microcarpa, E. mitchelliana, E. moluccana, E. moorei, E. morrisbyi, E. myriadena, E. neglecta, E. nicholii, E. niphophila, E. nitens, E. occidentalis, E. ovata, E. parvifolia, E. parvula, E. perriniana, E. petiolaris, E. piperita, E. plenissima, E. polyanthemos, E. polybractea, E. porosa, E. pulchella, E. pulverulenta, E. sideroxylon, E. risdonii, E. robusta, E. rodwayi, E. rostrata, E. rubida, E. sideroxylon, E. sieberi, E. staigeriana, E. subcrenulata, E. tenuiramis, E. tereticornis, E. tetraptera, E. tricarpa, E. urnigera, E. vegrandis, E. viminalis, E. viridis, E. youmanii,* and, insofar as such compounds are present, from *E. delegatensis, E. haemastoma, E. nitida, E. obliqua, E. pauciflora, E. radiata, E. regnans,* or *E. stellulata,* or from other plants from the genus Eucalyptus. Of course, it is also possible to use mixtures of such materials.

These plants originate from all over the world, mainly from Eastern Asia and Australia. They are used in traditional medicine e.g. in China and Australia.

The compounds of the formula I can be obtained by the extraction plants of plant material, e.g. of hackled parts of the plants as plant material, e.g. from leaves, bark, fruits, or further the stem,: roots or other parts of the plants. The method is characterized by an exhaustive extraction of fresh or dried plant material with an organic solvent, preferably an alcohol, optionally in the presence of water, and concentration to a crude extract. If desired, the crude extract is reextracted with one or more different organic solvents (especially those mentioned in the Examples for this reextraction) of ascending polarity, again followed by concentration. Such an advanced extract will optionally separated into the pure ingredients via ordinary purification methods e.g. chromatography or crystallisation.

It can be shown that these extracts are of different composition from that of the known essential oils, namely:
Group 1, Cineol (or Eucalyptol) containing oils, of which group *E*. *globulus* is the most important;
Group 2, Citronellal containing oils, of which group *E. maculata* is the most important;
Group 3, Citral containing oils, of which *E. staigeriana* F. von Muell., is the typical example;
Group 4, Peppermint smelling oils, of which *E. piperita* is an example;
and Group 5, Less known oils of varying odor.

These oils are prepared in a water steam driven process. This enriches low molecular weight compounds like monoterpenes e.g. pinen, eucalyptol and carvone, and sequiterpenes e.g. aromadendrene, gurjunene and globulol. No compounds of the formula I can be detected in these oils by coupled High Performance Liquid Chromatography/Mass Spectroscopy (HPLC-MS).

One embodiment of the invention therefore also relates to a method of producing a pharmaceutical or a nutraceutical product, comprising
a) extracting (especially as just described or more especially as described in the Examples) a compound of the formula I, or a mixture of compounds of the formula I, from a biological material, especially a plant or plant parts, preferably leaves and/or the bark, from the genus *Eucalyptus,* and
b) admixing the obtainable extract, the obtainable extracted compound of the formula I, or an obtainable extracted mixture of compounds of the formula I with further ingredients (especially any of the additives etc. as described above) to form a nutraceutical and/or a pharmaceutical composition.

The invention, in one embodiment, also comprises a nutraceutical or pharmaceutical composition, obtainable as described in the method of manufacture just given.

Stereoisomeric mixtures of a compound of the formula I, e.g. mixtures of diastereomers or cis/trans-isomers, as well as of starting materials can be separated into their corresponding isomers in a manner known per se by means of suitable separation methods. Diastereomeric mixtures or mixtures of cis/trans compounds for example may be separated into their individual diastereomers or cis/trans isomers by means of fractionated crystallization, chromatography (e.g. on silica gel, for example by thick layer chromatography), solvent distribution, and similar procedures. Enantiomers may be separated through the formation of diastereomeric salts, for example by salt formation with an enantiomer-pure chiral acid, or by means of chromatography, for example by HPLC, using chromatographic substrates with chiral ligands.

Fig. 1 shows an ¹H-NMR spectrum of the compounds named "unknown FPC" herein, obtained under conditions mentioned below in the Examples under "General Experimental Procedures".

### Examples

The following Examples serve to illustrate the invention without limiting the scope thereof: The embodiments shown therein may be adapted to variation in order to produce extracts embraced by this invention but not specifically disclosed. Further, variations of the methods to produce the same extracts in somewhat different fashion will be evident to one skilled in the art.

### General Experimental Procedures:

LC-MS analyses are performed using an Agilent HP1100 (Agilent, Waldbronn, Germany) liquid chromatograph coupled with a LCT mass spectrometer (Micromass, Manchester, UK) in the positive and negative electrospray ionisation (ESI) mode, based on slight modification of a previously described method [9]. A Waters symmetry column is used as stationary phase. Mobile phase A: 0.1 % Formic acid in water, mobile phase B: 0.1 % Formic acid in acetonitrile; gradient: 0-1 min. 100 % A, from 1-6 min. to 90 % B, from 6 to 8 min to 100 % B, from 8-10 min 100 % B. LC-MS spectra are recorded in the range of molecular weights between 150 and 1.600 U. HPLC-UV/Vis analyses are carried out on a HP 1100 Series analytical HPLC system (Agilent, Waldbronn, Germany) comprising a G 1312A binary pump system, a G 1315A diode array detector, a G 1316A column compartment, a G 1322A degaser and a G 1313A autoinjector. Mobile phase: A = 0.1 % Trifluoroacetic acid in water, B = 0.1 % Trifluoroacetic acid in acetonitrile. A (reversed phase) Macherey & Nagel (Düren, Germany) Nucleodur RP 18 column (125 x 4 mm, particle size 5 µm) serves as stationary phase. Aliquots of the samples (representing 2 - 10 µg of methanol-soluble materials, according to the concentrations of main metabolites) are analysed at 40°C with a flow of 1 ml/min in the following gradient: Linear from 0 % B to 100 % B in 20 min, thereafter isocratic conditions at 100% acetonitrile for 5 min; followed by regeneration of the column for 5 min. HPLC-UV chromatograms are recorded at 210 nm and 254 nm. Diode array detection (DAD) is employed to record HPLC-UV/Vis spectra in the range of 190 - 600 nm. The HP ChemStation software allows for an automated search for calibrated standard compounds in crude extracts. Preparative HPLC is performed at room temperature on a preparative HPLC system (Gilson Abimed, Ratingen, Germany), comprising Gilson Unipoint software, 306 binary pump system, 204 fraction collector, 155 UV-Vis detector, 806 manometric module, and 811C dynamic mixer, using different gradients and stationary phases as described below. NMR spectra are recorded on a Bruker DMX500, operating at 500.13 MHz proton frequency. All spectra are measured in DMSO-d₆ solution at 302 K. The solvent peak is used as internal reference for both proton and carbon chemical shifts (δ_{H}: 2.50, δ_{C}: 39.5).

### Example 1: extraction of mixture of meroterpenes:

2 kg of dried *Eucalyptus globulus* dry leaves (Mueggenburg Pflanzliche Rohstoffe GmbH & Co KG, Hamburg, Germany) are hackled and afterwards extracted three times with 10 L 95 % (v/v) methanol under vigorous stirring each for three hours at room temperature. The combined extracts are filtrated and reduced under vacuum in a rotary evaporator. Reextraction of the remaining water phase (approximately 900 mL) is performed first with 500 mL of petrol ether (PE), second with 250 mL of dichlormethane, third with 250 mL of ethyl acetate and finally with 200 mL butanol, each organic extraction is performed three times. The pooled ethyl acetate extracts are dried over sodium sulphate and evaporated under reduced pressure yielding 36,7 g crude product. (PE: 115 g, CH₂Cl₂: 102,1, butanol: 34,3 g, water residue: 72,5 g)

### Example 2: purification of compounds of the formula I:

The first purification step of the crude extract is performed on a (reversed phase) Nucleodur 100-5 C18ec column, 250 x 20 mm (Macherey & Nagel, Düren, Germany) using a linear water (Solvent A) / acenonitrile (Solvent B) gradient (both solvents contains 0,1 % trifluoroacetic acid) starting with 20 % acetonitrile ramped to 100 % acetonitrile in 50 minutes followed by an isocratic phase for 30 minutes. With a flow rate of 20 ml/min the fractions are collected each minute. Using a UV/VIS-155 detector (Gilson, Langenfeld, Germany) the signals are detected at 210 nm and 254 nm.

Eucalyptone: The linear gradient runs from 30 % B up to 100 % B in 50 minutes and continues additional 10 minutes. The yield of the desired product is 505 mg. The spectroscopic data comply with reported data. (Osawa, K. et al., Phytochemistry, 1995, 40, 183-184)

"Unknown FPC" is also isolated by HPLC and shows the ¹H-NMR spectrum given in Fig. 1.

### Example 3: cell based assay

### Materials:

| | |
|---|---|
| 5-[1,2-³H(N)]-Hydroxytryptamin-creatininsulfate (5-HT) | NET-498, Lot 3499-250 (ligand) |
| levo-[ring-2,5,6-³H]-Norepinephrine (NA) | NET-678, Lot 3557477 (ligand) |
| 3,4-[ring-2,5,6-³H]-Dihydroxyphenylethylamine (DA) | NET-637, Lot 3557411 (ligand) |
| Fluvoxamine | Sigma F 2802, Lot 71 K4702 |
| Desipramine hydrochloride | Sigma D 3900, Lot 064K1370 |
| Nomifensine maleate | Sigma N1530, Lot 095K4064 |
| Dopamine hydrochloride | Fluka 56610, Lot 361042/1 51597 |
| Norepinephrine bitartrate | Sigma A 9512, Lot 100 K 1484 |
| HEPES | Sigma H 4034, Lot 81 K5418 |
| Ultima Gold | Packard Nr. 6013329, Charge: 77-060201 |

Other materials are of appropriate grade as commonly commercially available.

Assay description (see also general description on the transport process of neurotransmitters in rat synaptosomes):
Male wistar rats (200-300g, 8-12 weeks old) are decapitated under CO₂ anaesthesia and brains are quickly removed. Neocortex tissue is immediately immersed in 10 volumes of icecold 0,32 M sucrose buffered with 10 mM HEPES pH 7,4 and homogenized with a Potter-Elvehjem glass/teflon homogenizer at 500 rpm (IKA, Müllheim, Germany). The resulting preparation is centrifuged at 900 x g for 10 minutes at 4 °C. The pellet is discarded and the supernatant centrifuged again at 4 °C at 10.000 x g (Biofuge 28RS, Heraeus, Hanau, Germany). The supernatant is discarded and the pellet is stored with 0.32 M sucrose/HEPES on ice until needed.

Assays are carried out in Farnebo buffer pH 7,4 (121 mM NaCl, 1,8 mM KCl, 1,3 mM CaCl₂, 1,2 mM MgSO₄, 25 mM NaHCO₃, 1,2 mM KH₂PO₄, 11 mM glucose, 0,57 mM ascorbic acid, saturated with 95% O₂/5% CO₂) containing the MAO inhibitor pargyline in a concentration of 50 µM. The pellet containing synaptosomes was resuspended in Farnebo buffer at a protein concentration of about 1 mg/ml.

Experiments are carried out in 96 well filtration plates (Multiscreen, Millipore, Eschborn, Germany). For each plate the control uptake and the non-specific uptake (in the presence of of 10 µM fluvoxamine (5-HT), 10 µM desipramine (NA) or 10 µM nomifensine (DA)) are determined. Each drug concentration is measured in 4 wells. For one plate the same synaptosome preparation is used.

10 µl of drug solution in DMSO, non-specific ligand or buffer, 180 µl Farnebo buffer and 50 µl of synaptosome preparation resuspended in Farnebo buffer are added to each well of a 96 well filtration plate prewetted with Farnebo buffer. The incubation proceeds for 10 minutes at room temperature after which 10 µl of [³H]-ligand (final concentrations: 5-HT: 2 nM, NA: 20 nM, DA: 30 nM) are added to a total volume of 250 µl. The incubation proceeds for 15 minutes (DA, NA) or 20 min (5-HT) at room temperature. Incubation is terminated by rapid filtration and washing with Farnebo buffer. Radioactivity remaining on the filters is estimated with a liquid scintillation counter with an efficiency of about 50%. Specific binding is defined as total binding minus binding in the presence of 10 µM fluvoxamine (5-HT), 10 µM desipramine (NA) or 10 µM nomifensine (DA).

| EC₅₀ values [µg/ml] compound | Dopamine | Norepinephrine | Serotonine |
|---|---|---|---|
| GTG 183-1-1 | 10 | 13 | 31 |
| GTG 184-1-1 | 4 | 4 | 17 |

| | | | |
|---|---|---|---|
| GTG 183-1-1: extract prepared according example 1 GTG 184-1-1: mixture of pure Eucalyptone and "unknown FPC" 1:1 by weight. | | | |

### Example 4: pharmaceutical composition

The following ingredients are used for the preparation of 5000 tablets each containing 200 mg of active ingredient (an extract according to Example 1 or a 1:1 mixture of pure Eucalyptone and Macrocarpale B):

| | |
|---|---|
| active ingredient | 1000 g |
| corn starch | 680 g |
| colloidal silica | 200 g |
| magnesium stearate | 20 g |
| stearic acid | 50 g |
| sodium carboxymethyl starch | 250 g |
| water | quantum satis |

A mixture of one of the compounds of formula I mentioned in the preceding Examples (e.g. Example 1) as active ingredient, 50 g of corn starch and the colloidal silica is processed with a starch paste, made from 250 g of corn starch and 2.2 kg of demineralised water, to form a moist mass. This is forced through a sieve having a mesh size of 3 mm and dried at 45° for 30min in a fluidised bed drier. The dry granules are pressed through a sieve having a mesh size of 1 mm, mixed with a pre-sieved mixture (1 mm sieve) of 330 g of corn starch, the magnesium stearate, the stearic acid and the sodium carboxymethyl starch, and compressed to form slightly biconvex tablets.

## Claims

1. A compound of the formula I or a mixture of compounds of the formula I, 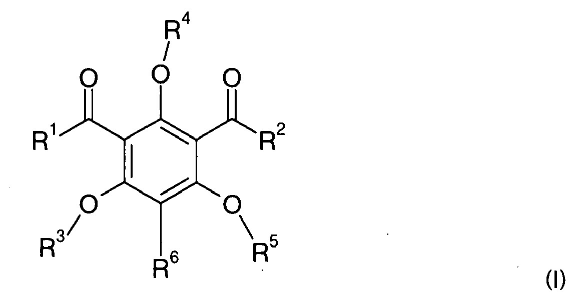 wherein
R¹ and R² are, independently of each other, hydrogen or linear or branched C₁-C₁₀-alkyl;
R³ and R⁴ are, independently of each other, hydrogen or linear or branched C₁-C₁₀-alkyl;
R⁵ is hydrogen or linear or branched C₁-C₁₀-alkyl; and
R⁶ is linear or branched C₁-C₁₂-alkyl that is itself substituted with C₃-C₂₅-hydrocarbyl with at least one cyclic element that is saturated or includes one or more isolated - not conjugated or cumulated - double and/or triple carbon-carbon bonds and that is unsubstituted or substituted by one or more substituents independently selected from the group consisting of hydroxyl, linear or branched C₁-C₅-alkyl, linear or branched C₁-C₅-alkyliden, (linear or branched C₁-C₅-alkyl)oxy, hydroxyl-C₁-C₅-alkyl, oxo, linear or branched C₁-C₁₀alkyl substituted with oxo and cycloalkyl with 3 to 10 carbon atoms moieties, the cycloalkyl itself unsubstituted or substituted with one or more substituents selected from hydroxyl, linear or branched C₁-C₅-alkyl, linear or branched C₁-C₅-alkyliden, (linear or branched C₁-C₅-alkyl)oxy, hydroxyl-C₁-C₅-alkyl, oxo and linear or branched C₁-C₁₀alkyl substituted with oxo;
or one of R³ and R⁵ is hydrogen or linear or branched C₁-C₁₀-alkyl, the other is a bond to the C₃-C₂₅-hydrocarbyl part of the C₁-C₁₂-alkyl substituted with unsubstituted or substituted C₃-C₂₅-hydrocarbyl as defined above for R⁶ which thus, bound via a first bond to the O to which R³ or R⁵ is bound and via a second bond to the C₁-C₁₂-alkyl part of R⁶, forms a C₃-C₂₅-hydrocarbondiyl with at least one cyclic element that is saturated or includes one or more isolated - not conjugated or cumulated - double and/or triple carbon-carbon bonds and that is unsubstituted or substituted by one or more substituents independently selected from the group consisting of hydroxyl, linear or branched C₁-C₅-alkyl, linear or branched C₁-C₅-alky-liden, (linear or branched C₁-C₅-alkyl)oxy, hydroxyl-C₁-C₅-alkyl, oxo, linear or branched C₁-C₁₀alkyl substituted with oxo, and cycloalkyl with 3 to 10 carbon atoms moieties, the cycloalkyl itself unsubstituted or substituted with one or more substituents selected from hydroxyl, linear or branched C₁-C₅-alkyl, linear or branched C₁-C₅-alkyliden, (linear or branched C₁-C₅-alkyl)oxy, hydroxyl-C₁-C₅-alkyl, oxo and linear or branched C₁-C₁₀alkyl substituted with oxo, which C₃-C₂₅-hydrocarbondiyl thus together with the O binding R³ or R⁵ in formula I, the carbon binding said O and the carbon binding R⁶ in formula I, the bond connecting these two carbons and the C₁-C₁₂-alkyl bound in the position of R⁶ forms a further ring,
with the proviso that the carbohydryl and the alkyl part of R⁶ together have at least 10 carbon atoms,
and/or a tautomer or tautomers, a solvate or solvates, an ester or esters and/or a salt or salts thereof,
for use as central nervous system (CNS) activity modulators in the prophylactic and/or therapeutic treatment of an individual.

2. A compound or compounds of the formula I for use according to claim 1, where the compound or compounds are selected from those wherein
R¹ and R² are, independently of each other, hydrogen or linear or branched C₁-C₁₀-alkyl;
R³ and R⁴ are, independently of each other, hydrogen or linear or branched C₁-C₁₀-alkyl;
R⁵ is hydrogen or linear or branched C₁-C₁₀-alkyl;
R⁶ is linear or branched C₁-C₁₂-alkyl that is itself substituted with C₃-C₂₅-hydrocarbyl which is selected from the group consisting of ring systems with the following formulae, the waved line marking the end of the bond at the preferred place of binding to the linear or branched C₁-C₁₂-alkyl, which is preferably methyl or ethyl, or is isopentyl substituted by hydrocarbyl at the C¹, that is, isopentan-1,1-diyl, substituted by said hydrocarbyl with which it forms R⁶: 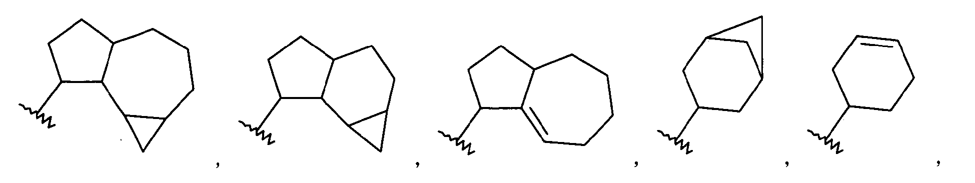 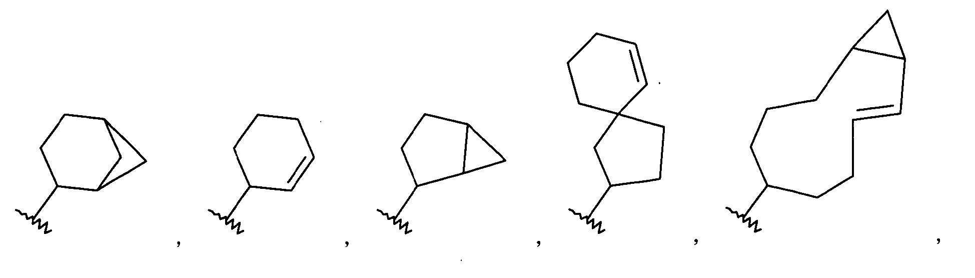 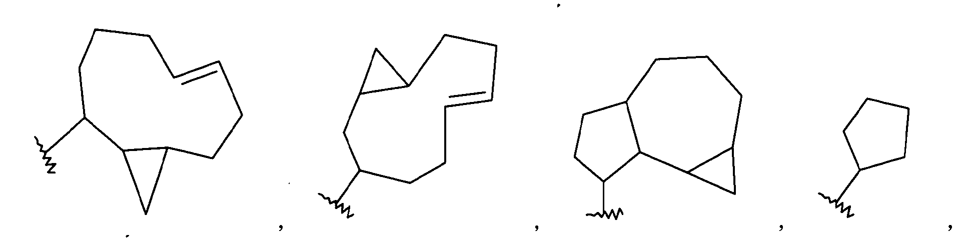 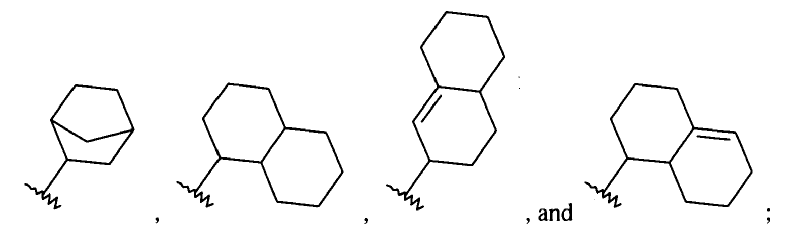 or one of R³ and R⁵ is hydrogen or linear or branched C₁-C₁₀-alkyl, the other is a bond to the C₃-C₂₅-hydrocarbyl part of the C₁-C₁₂-alkyl substituted with C₃-C₂₅-hydrocarbyl as defined above for R⁶ which thus, bound via a first bond to the O to which R³ or R⁵ is bound and via a second bond to the C₁-C₁₂-alkyl part of R⁶, forms a C₃-C₂₅-hydrocarbondiyl which C₃-C₂₅-hydrocarbondiyl together with the O binding R³ or R⁵ in formula I, the carbon binding said O and the carbon binding R⁶ in formula I, the bond connecting these two carbons and the C₁-C₁₂-alkyl bound in the position of R⁶ forms a further ring, and which C₃-C₂₅-hydrocarbondiyl is selected from the group consisting of ring systems with the following formulae, the waved line marking the end of the bond of the preferred place of binding to the linear or branched C₁-C₁₂-alkyl, preferably defined as given above the preferred formulae for hydrocarbyl R⁶, and the asterisk marking the preferred end of the bond where the O that binds R³ or R⁵ in formula I is bound: 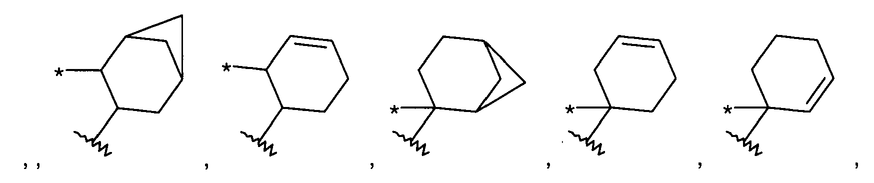 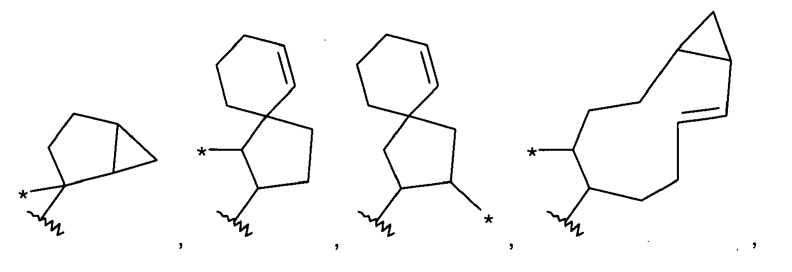 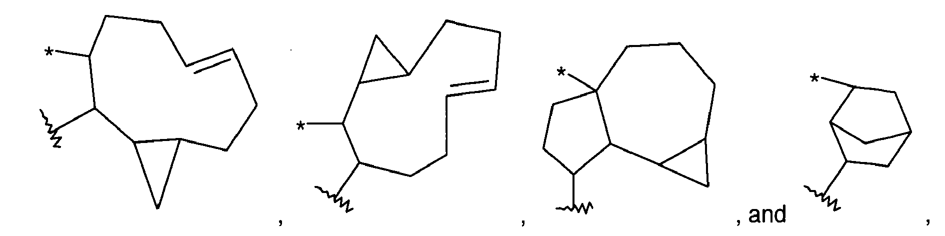 where each C₃-C₂₅-hydrocarbyl and/or C₃-C₂₅-hydrocarbondiyl is unsubstituted or substituted by one or more, especially up to five, substituents independently selected from the group consisting of hydroxyl, linear or branched C₁-C₅-alkyl, linear or branched C₁-C₅-alkyliden, (linear or branched C₁-C₅-alkyl)oxy, hydroxyl, hydroxyl-C₁-C₅-alkyl, oxo, linear or branched C₁-C₁₀alkyl substituted with oxo, and cycloalkyl with 3 to 10 carbon atoms moieties, the cycloalkyl itself unsubstituted or substituted with one or more substituents selected from hydroxyl, linear or branched C₁-C₅-alkyl, linear or branched C₁-C₅-alkyliden, (linear or branched C₁-C₅-alkyl)oxy, hydroxyl, hydroxyl-C₁-C₅-alkyl, oxo and linear or branched C₁-C₁₀alkyl substituted with oxo;
with the proviso that the carbohydryl and the alkyl part of R⁶ together have at least 10 carbon atoms,
and/or a tautomer or tautomers, a solvate or solvates, an ester or esters and/or a salt or salts thereof.

3. A compound or mixture of compounds of formula I for use according to claim 1 or claim 2 which is an extract, preferably wherein the compound or compounds of the formula I are present totally in a purity of 20 to 100 %, more preferably from 50 to 100 % by weight.

4. A compound or mixture of compounds of formula I for use according to claim 3 which is an extract from a plant or plant material of the Genus *Eucalyptus* and/or from a Microorganism of the genus *Pseudomonas.*

5. A compound or mixture of compounds of the formula I for use according to claim 1 in the form of an extract according to any one of claims 3 or 4 obtainable by a method of manufacture comprising extracting plant material from plants of the Genus *Eucalyptus,* especially hackled parts of the plants, more especially bark, fruits, stems, roots or especially leaves, with an organic solvent, preferably an alcohol, in the absence or presence of water; and, if desired, reextracting the crude extract with one or more different solvents of ascending polarity; and, if desired, further purifying the obtainable extract, especially by chromatography and/or crystallisation.

6. A compound or mixture of compounds according to any one of claims 1 to 5, where the compound or compounds is or are selected from the group of compounds with the following names: Macrocarpal B, Macrocarpal C, Macrocarpal A, Antibiotic GR 95647X, Macrocarpal G, Euvimal 1, Macrocarpal D, Euglobal G1, Euglobal G2, Euglobal G8, Euglobal G9, Euglobal G10, Euglobal G11, Euglobal Ia₁, Euglobal Ia₂, Euglobal IIc, Euglobal IIc Δ7-Isomer, Euglobal T1, Euglobal T1 Δ7-Isomer, Euglobal G4, Euglobal G3, Euglobal G12, Euglobal IIb, Robustadial A, Euglobal Am 1, Robustadial A 7-Epimer, Euglobal Am 2, Euglobal Ic, Euglobal IIa, Euglobal BI 1, Euglobal Ib, Euglobal In 1, Euglobal In 2, Euglobal In 3, Euglobal III, Euglobal IVb, Euglobal IVa, Euglobal VII, Euglobal V, Eucalyptone, Euglobal G5, Macrocarpal I, Macrocarpal J, Epimer of Macrocarpal I, Macrocarpal D, Macrocarpal E, Macrocarpal H and Macrocarpal K, and/or a tautomer or tautomers, a solvate or solvates, an ester or esters and/or a salt or salts thereof.

7. A compound or mixture of compounds of the formula I for use according to claim 1, where the compound or compounds are selected from Eucalpytone and an aylated phloroglucinol having the ¹H-NMR spectrum given in Fig. 1, or an ester or esters, a solvate or solvates and/or a salt or salts thereof.

8. A compound or mixture of compounds of the formula I according to claim 1, where the use is in prophylactically and/or therapeutically lifting the mood, increasing cognitive performance, positively affecting acquisition of memory, enhancing working memory, positively affecting motivation, positively influencing behavioural initiative and/or increasing general quality of living.

9. The use of a compound of the formula I, or a mixture of compounds of the formula I, where the compounds or compound mixtures are as defined in any one of claims 1 to 7, and/or a tautomer or tautomers, a solvate or solvates, an ester or esters and/or a salt or salts thereof, in the manufacture of a pharmaceutical composition or a nutraceutical for the treatment and/ or prophylaxis of depression, depressive disorders, anxiety and affective disorders, especially where one or more of the following symptoms or disorders is present and/or to be avoided prophylactically: general anxiety disorders, fear, cognitive disorders, learning and memory deficits and dysfunctions, manic depression, obsessive compulsive disorders, panic disorders, eating disorders, narcolepsy, sleeping disorders, such as chronic fatigue syndrome and jet lag, lack of ability to make decisions, lack of concentration, inferiority feeling, lethargy, anergy, lack of drive, dysphoria, melancholy, diminished emotional well-being, mood depresssion, low spirits, inner restlessness, feelings of being guilty, delusion of being guilty, stiff thinking, inhibition of thinking, circular way of thinking, feeling of helplessness, feeling of hopelessness, lack of appetite, enlarged susceptibility to infection due to a depressed mood, decreased cognitive abilities and/or other symptoms, as well as for mood-lifting in healthy persons, and/or also in the treatment of schizophrenia or psychotic diseases.

10. A pharmaceutical or nutraceutical composition comprising a compound of the formula I, or a mixture of compounds of the formula I, and/or a tautomer or tautomers, a solvate or solvates, an ester or esters and/or a salt or salts thereof , as shown in any one of claims 1 to 7, as active ingredient together with a pharmaceutically acceptable diluent or carrier for use in the therapeutic and/or prophlylactic treatment of one or more diseases or disorders selected from the group consisting of depression, depressive disorders, anxiety and affective disorders, especially where one or more of the following symptoms or disorders is present and/or to be avoided prophylactically: general anxiety disorders, fear, cognitive disorders, learning and memory deficits and dysfunctions, manic depression, obsessive compulsive disorders, panic disorders, eating disorders, narcolepsy, sleeping disorders, such as chronic fatigue syndrome and jet lag, lack of ability to make decisions, lack of concentration, inferiority feeling, lethargy, anergy, lack of drive, dysphoria, melancholy, diminished emotional well-being, mood depresssion, low spirits, inner restlessness, feelings of being guilty, delusion of being guilty, stiff thinking, inhibition of thinking, circular way of thinking, feeling of helplessness, feeling of hopelessness, lack of appetite, enlarged susceptibility to infection due to a depressed mood, decreased cognitive abilities and/or other symptoms, as well as for mood-lifting in healthy persons, and/or also in the treatment of schizophrenia or psychotic diseases.
